# EUROPEAN PATENT SPECIFICATION

(11) **EP 2 455 030 B1**
(45) Date of publication and mention of the grant of the patent: **27.01.2016**
(21) Application number: 11190169.0
(22) Date of filing: 22.11.2011
(51) Int. Cl.: A61B 17/70, A61B 17/88

(54) **Crimping tool and method for manufacturing a polyaxial bone anchoring device**
Crimpwerkzeug und Verfahren zur Herstellung einer polyaxialen Knochenverankerungsvorrichtung
Outil de sertissage et procédé pour la fabrication d'un dispositif d'ancrage d'os polyaxial

(30) Priority: 22.11.2010 US 416137 P; 04.03.2011 US 449349 P; 22.11.2010 EP 10192079
(43) Date of publication of application: 23.05.2012
(73) Proprietor: Biedermann Technologies GmbH & Co. KG, 78166 Donaueschingen (DE)
(72) Inventor: Biedermann, Lutz, 78048 VS-Villingen (DE); Pohl, Gerhard, 78112 St. Georgen (DE)
(74) Representative: Prüfer & Partner mbB European Patent Attorneys

(56) References cited:
- WO-A1-2010/103198
- FR-A1- 2 684 866
- US-A- 5 558 674
- US-A- 5 672 176
- US-A- 5 681 319
- US-A1- 2005 049 589
- US-A1- 2010 137 920

## Description

The invention relates to a crimping tool for producing a polyaxial bone anchoring device for anchoring a stabilization rod in a bone or in a vertebra. The crimping tool comprises tongs with ends in form of handles and claws for gripping a receiving part of a polyaxial bone anchoring device, wherein the claws comprise at least one crimping.

US 5,716,356 describes a polyaxial bone screw including a screw element and a receiving part which is pivotably connected to the screw element and a pressure element to exert pressure onto the head of the screw element to lock the angle between the screw element and the receiving part. The receiving part has a U-shaped channel for receiving a stabilization rod. The pressure element comprises a cylindrical recess, which is to be aligned with the U-shaped channel to receive the rod therein. In order to hold the pressure element in a position aligned with the U-shaped channel, the position of the pressure element is fixed by crimping through bores provided in the receiving part.

When the head of the bone anchoring element is freely pivotable with respect to the receiving part before locking the head in a final angular position, the alignment of the receiving part and the insertion of the rod may be difficult in more complex clinical applications, for example, when a multitude of bone anchors have to be connected to the rod.

US 7,604,656 describes a fastener engageable with a bone portion to connect a longitudinal member to the bone portion. The housing that receives the fastener also receives a spacer, which is engageable with the fastener and the longitudinal member. In one embodiment, the spacer is urged by a pin member into frictional engagement with the fastener and with the housing.

US 2004/0267264 A1 describes a polyaxial fixation device, wherein the polyaxial bone screw includes an engagement member that is adapted to provide sufficient friction between the spherical head and the receiver member to enable the shank to be maintained in a desired angular orientation before locking the spherical head within the receiver member. The engagement member is realized, for example, by a snap ring around the head or by spring members provided at the compression cap to frictionally engage the spherical head or by a slot provided in the compression cap.

US 2005/049589 A1 discloses a generic crimping tool. Another tool is known from US 5,558,674 A.

It is the object of the invention to provide a method for manufacturing a polyaxial bone anchoring device which allow an improved handling during surgery.

This object is achieved by the methods having the features of claims 1 and 2. The invention is further developed as defined in the dependent claims.

With the polyaxial bone anchoring device a temporary clamping of the head in a desired angular position with respect to the receiving part without locking the head can be achieved. This allows to maintain the receiving part in an adjustable angular position. In this condition, the pressure element exerts a preload onto the head in which the head is not locked but prevented from freely pivoting. When the head is temporarily clamped, the alignment of the receiving part with respect to the rod and the insertion of the rod is facilitated, in particular in a situation in which a multitude of bone anchors have to be connected to the rod.

When the rod is already inserted into the receiving part, adjustments of the rod are still possible without completely loosening the head.

The polyaxial bone anchoring device comprises only few parts which are of simple design. The mechanism to frictionally maintain the head before locking it is free from any spring members or portions. This facilitates the manufacturing of the polyaxial bone anchoring device. Further-more, existing receiving parts and pressure elements can be used without having to redesign their shape. It is possible to simply change the location of the crimp bores.

The amount of preload exerted onto the head by the pressure member can be exactly predefined in a simple manner by selecting the position and shape of the crimp bores. The polyaxial bone anchoring device is provided to the surgeon in a pre-assembled manner, in which the pressure element is axially and rotationally fixed to such an extent that it can not fall out or be rotated out of its aligned position. This allows a safe handling by the surgeon.

The receiving part and the pressure element can be manufactured in series at low costs.

Further features and advantages of the invention will become apparent from the description of embodiments by means of the accompanying drawings.

In the drawings:
- Fig. 1: shows a perspective exploded view of the polyaxial bone anchoring device according to a first embodiment.
- Fig. 2: shows the polyaxial bone anchoring device of Fig. 1 in an assembled state.
- Fig. 3: shows a cross-sectional view of the polyaxial bone anchoring device in an assembled state before final locking of the head.
- Fig. 4a: shows a cross-sectional view of the polyaxial bone anchoring device before provisionally fixing the pressure element in the receiving part.
- Fig. 4b: shows an enlarged portion of Fig. 4a.
- Fig. 5a: shows a cross-sectional view of the polyaxial bone anchoring device in the pre-assembled state after provisionally fixing of the pressure element in the receiving part.
- Fig. 5b: shows an enlarged portion of Fig. 5a.
- Fig. 6: shows a cross-sectional view of a tool for provisionally fixing the pressure element in the receiving part.
- Fig. 7: shows an enlarged portion of Fig. 6.
- Fig. 8: shows a cross-sectional view of a modified embodiment of the polyaxial bone anchoring device before locking of the head.
- Fig. 9: shows a cross-sectional view of a further modified embodiment of the polyaxial bone anchoring device in a state before locking of the head.
- Fig. 10: shows a perspective exploded view of a second embodiment of the polyaxial bone anchoring device.
- Fig. 11: shows an enlarged cross-sectional view of a portion of the pressure element of the bone anchoring device of Fig. 10.
- Fig. 12: shows a cross-sectional view of the polyaxial bone anchoring device of the second embodiment before provisionally fixing the pressure element in the receiving part.
- Fig. 13: shows a cross-sectional view of the polyaxial bone anchoring device of Fig. 12 in the pre-assembled state after provisionally fixing of the pressure element in the receiving part.
- Fig. 14: shows a perspective exploded view of a further crimping tool that can be hand-operated.
- Fig. 15: shows a perspective view of the crimping tool of Fig. 14 in an assembled state.
- Fig. 16: shows an enlarged perspective view of a portion of the crimping tool of Figs. 14 and 15 with claws in an opened state and the polyaxial bone anchoring device not yet inserted.
- Fig. 17: shows an enlarged perspective view of a portion of the crimping tool of Fig. 14 and 15 with the polyaxial bone anchoring device inserted and the claws not yet fully closed.
- Fig. 18: shows an enlarged perspective view of a portion of the crimping tool of Fig. 14 and 15 with the polyaxial bone anchoring device inserted and the claws closed.
- Fig. 19a): shows a sectional view of a portion of the crimping tool of Fig. 14 and 15 with the polyaxial bone anchoring device inserted and the claws not yet closed, the section being taken perpendicular to the rod axis of the polyaxial bone anchoring device.
- Fig. 19b): shows an enlarged sectional view of a portion of Fig. 19a).
- Fig. 20a): shows a sectional view of a portion of the crimping tool of Fig. 14 and 15 with the polyaxial bone anchoring device inserted and the claws closed, the section being taken perpendicular to the rod axis of the polyaxial bone anchoring device.
- Fig. 20b): shows an enlarged cross-sectional view of a portion of Fig. 20a).

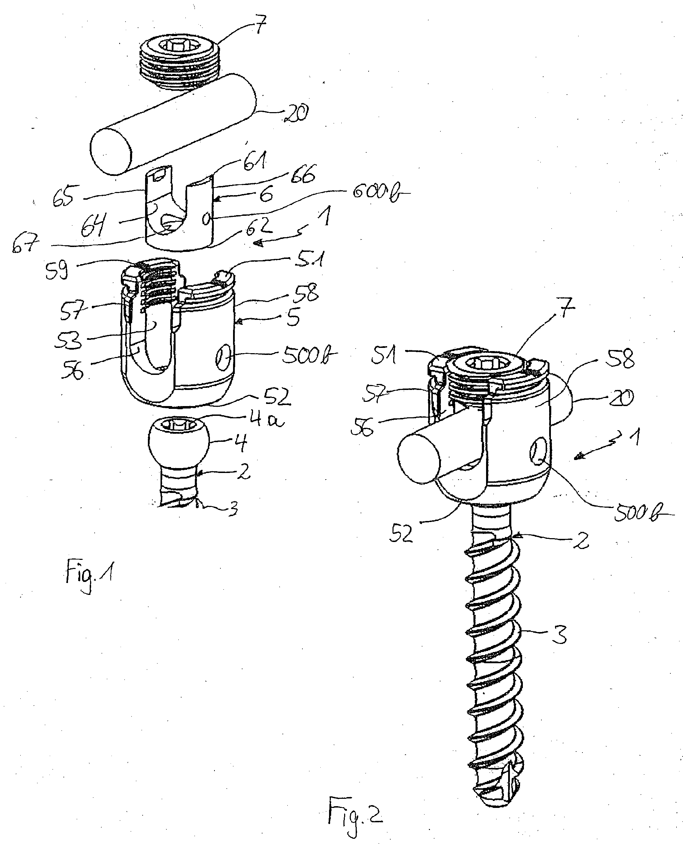

The polyaxial bone anchoring device 1 according to a first embodiment as shown in Figs. 1 to 3 includes a bone anchoring element in the form of a screw member 2 having a threaded shaft 3 and a head 4. The head 4 is generally spherical and includes a recess 4a at its free end for engagement with a tool to insert the threaded shaft 3 into bone. The bone anchoring device further includes a receiving part 5 for connecting the screw member 2 to a rod 20. A pressure element 6 is arranged in the receiving part on top of the head 4. For securing the rod 20 in the receiving part and for exerting pressure onto the head, a locking device, for example an inner screw 7, which cooperates with the receiving part 5, is provided.

The receiving part is a substantially cylindrical one piece part and has a top end 51 and a bottom end 52. A passageway extending from the top end to the bottom end is formed by a coaxial bore 53 followed by a seat portion 54 for receiving the head 4 of the screw member 2. The seat portion 54 has an opening 55 at the bottom end 52 through which the shaft 3 of the screw member extends. The seat portion 54 is shown to be spherically-shaped, but it can be tapered or it can have any other shape that allows to receive the head 4 so that it can pivot with respect to the receiving part 5. At the top end 51 a substantially U-shaped recess 56 is provided by means of which two free legs 57, 58 are formed that are the sidewalls of a channel for receiving the rod 20. An internal thread 59 is provided at the legs for cooperating with the inner screw 7.

The pressure element 6 is formed in one piece. It is of substantially cylindrical construction and has an outer diameter, which allows it to move in the axial direction within the bore 53 of the receiving part 5. The pressure element 6 has a top end 61 and a bottom end 62. When the pressure element is inserted into the receiving part, the bottom end 62 faces the head 4 of the screw element 2. At the bottom end 62 a spherical recess 63 is provided, which is adapted to the size and shape of the head 4. The spherical recess is configured to come into frictional engagement with the spherical surface of the head. At the top end 61, a U-shaped recess 64 is provided by means of which two free legs 65, 66 are formed that form a channel to receive the rod 20 therein. Furthermore, the pressure element 6 includes a coaxial bore 67 for accessing the screw head 4 with a tool (not shown). As shown in the Figs., the pressure element 6 is a solid member without any spring portions, which could render it flexible. It is arranged in the receiving part such that the U-shaped recess 56 of the receiving part 5 and the U-shaped recess 64 of the pressure element are aligned.

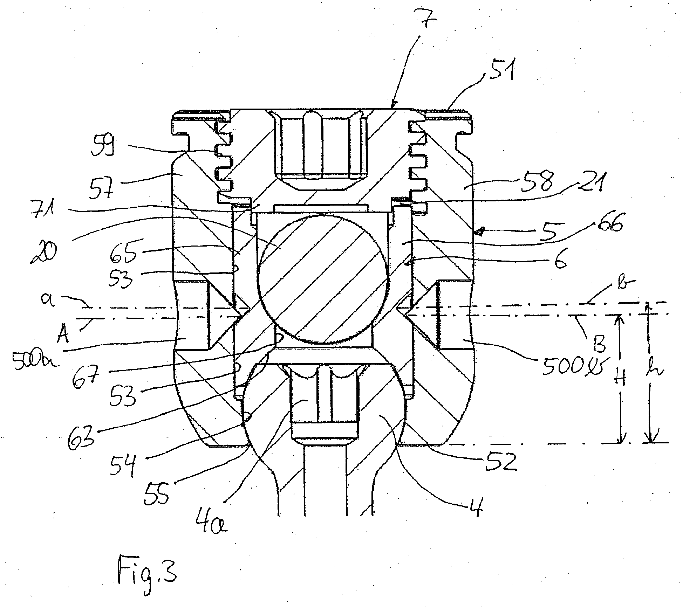

In the assembled state as shown in Fig. 3, the screw head 4 is located in the seat 54 and the pressure element 6 is arranged on top of the screw head 4. The height of the free legs 65, 66 of the pressure element is configured such that the free legs 65, 66 extend above the rod 20 when the rod is inserted and rests on the bottom of the channel.

The locking device in the form of the inner screw 7 has a projection 71 extending into the channel formed by the free legs 65, 66 of the pressure element 6. The size of the projection 71 in an axial direction is such that when the inner screw 7 is tightened, the projection 71 presses onto the rod while there is still a gap 21 between the top end 61 of the pressure element and the lower side of the inner screw 7. Therefore, with the single inner screw 7 pressure can be exerted onto the rod 20 only, which in turn can exert pressure onto the pressure element 6. It should be noted that instead of the single part locking device in form of the inner screw 7 a two-part locking device can be used (not shown). The two-part locking device includes a first part to be screwed in-between the legs 57, 58 of the receiving part. The first part acts onto the top end 61 of the pressure element 6. Further, a second part in form of an inner screw is provided in the first part, which presses onto the rod 20. By means of this, the head 4 and the rod 5 can be independently fixed.

The pressure element 6 is retained in the receiving part 5 as shown in Figs. 3 to 5. As shown in particular in 4a and 4b, the receiving part includes two blind holes 500a, 500b forming crimp bores that extend from the outer surface to a distance from the inner wall of the coaxial bore 53. The blind holes 500a, 500b are arranged at 180° offset from each other and at 90° with respect to the channel formed by the U-shaped recess 56. The blind holes 500a, 500b are aligned perpendicular with respect to the bore axis M of the coaxial bore 53. At their end they are tapered with an angle α preferably less than 45°, for example 22,5°, with respect to an axis parallel to the bore axis M. The bore axes A and B of the blind holes 500a, 500b are provided at a distance H from the second end 52 of the receiving part.

The portions of the receiving part that are between the closed ends of the blind holes 500a, 500b and the coaxial bore 53 of the receiving part are configured to be deformable portions 501a, 501b.

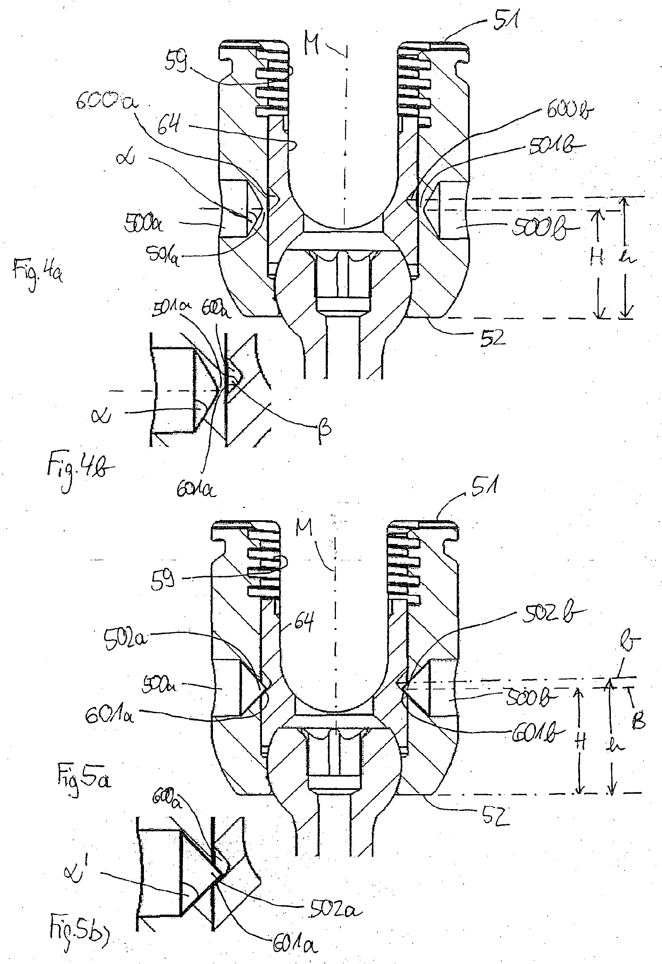

The pressure element 6 correspondingly includes two recesses 600a, 600b which are 180° offset from each other and 90° offset from the channel formed by the U-shaped recess 64. The recesses 600a, 600b have a center axis a, b, respectively, which is perpendicular to the bore axis M. In the embodiment shown, the recesses 600a, 600b have a conical shape. The downwardly extending flanks 601 a, 601 b of the recesses 600a, 600b each include an angle β of approximately 45° with the central bore axis M. As shown in Fig. 4a and 4b, when the pressure element 6 is inserted such that it rests on the head 4 of the screw element, the central axis a, b of the recesses 600a, 600b has a distance h from the second end 52 of the receiving part 5 that is greater than the distance H of the central axis A, B of the blind holes 500a, 500b. In other words, the recesses 600a, 600b are arranged above the blind holes 500a, 500b.

The distance between the recesses and the blind holes is such that when the deformable portions 501a, 501b are deformed by applying a force via for example, a crimping tool to the blind holes 500a, 500b, the deformed material protrudes from the inner wall of the receiving part and presses onto the lower flanks 601a, 601b of the recesses 600a, 600b, respectively, to exert a downward force onto the pressure element 6. As shown in Figs. 5a and 5b, deforming the deformable portions 5a, 5b results in deformed portions 502a, 502b which exert pressure on the lower flank 601a, 601b of the recesses 600a, 600b of the pressure element 6. For example, after deformation, the angle α is approximately 45°, which is approximately the same as the angle of the lower flank 601a, 601b. The blind holes 500a, 500b with their respective deformable portions 501 a, 501 b and the recesses 600a, 600b are constructed such that by deforming the deformable portions 501a, 501b into deformed portions 502a, 502b which engage the recesses 600a, 600b, the resulting force onto the pressure element 6 generates a preload onto the head 4, which clamps the head by means of friction. By selecting the sizes of blind holes and the recesses and their position, a desired friction force can be achieved. By this friction force the head can be maintained in a desired angular position and can be moved out of this position by applying a force greater than the friction force either to the screw element or to the receiving part. Simultaneously, the pressure element is secured against rotation and secured against escaping through the top end 51 of the receiving part. The recesses 600a, 600b provide space for accommodating a part of the deformed material. Also, the recesses 600a, 600b provide space for the protrusions 502a, 502b when the pressure element 6 moves downward to finally lock the head.

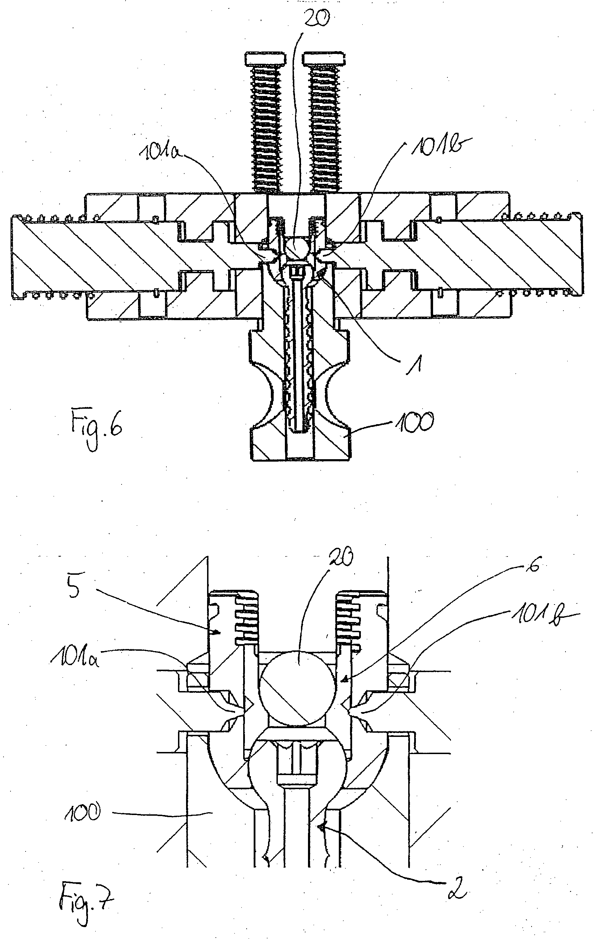

A method for manufacturing the polyaxial bone anchoring device is explained with reference to Figs. 6 and 7 and 4 to 5. A crimping tool shown in Figs. 6 and 7 generally comprises a holder 100 for the bone anchoring device, which serves for fixing the receiving part 5 with inserted screw element 2 and pressure element 6 as it is shown in Figs. 4a and 4b. The rod 20 may be inserted for providing a counter-force to avoid deformation of the free legs 65, 66 of the pressure element. The crimping tool further includes two crimping tips 101a, 101b, which are arranged 180° offset from each other and are dimensioned to be introduced into the blind holes 500a, 500b and to deform the deformable portions 501a, 501b, so that the displaced material, which forms the deformed portions 502a, 502b, engages the recesses 600a, 600b of the pressure element. As can be seen in particular in Fig. 7, the crimping tips 101a, 101b have an angle, which is more acute than that of the bottom of the blind hole 500a, 500b. The crimping tips 101a, 101b deform the deformable portion such that the deformed portion presses onto the lower flank 601a, 601b of the recess 600a, 600b, respectively. Thereafter, the crimping tips are retracted. The crimping process can be force-actuated and/or path-controlled.

After the crimping tips are retracted, the polyaxial anchoring device can be removed from the holder 100. The polyaxial bone anchoring device is then in a pre-assembled state with the screw element 2 being inserted and the pressure element being held in such a way that it exerts a slight preload onto the head which frictionally holds the head in an angular position.

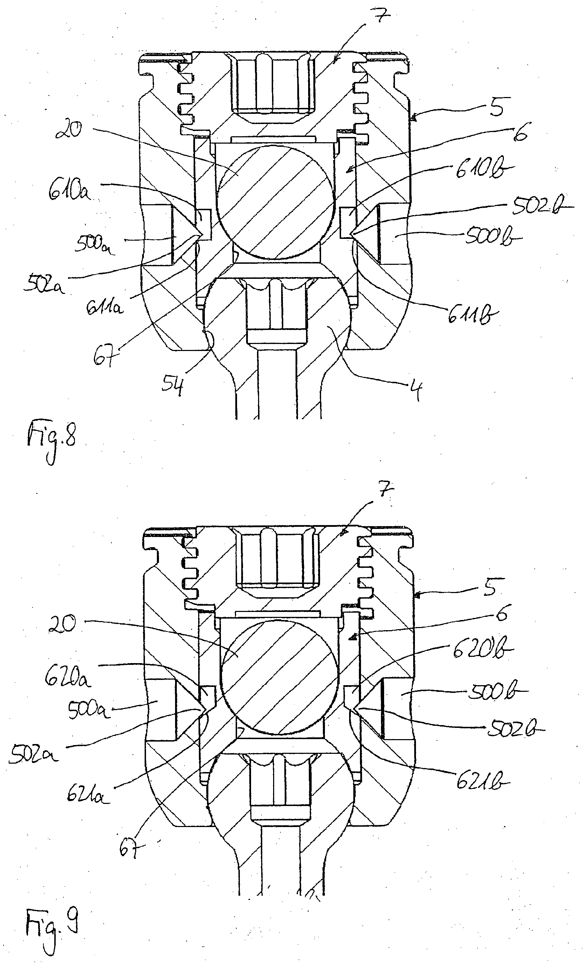

It shall be noted that the shape of the blind holes may vary. In particular, the angle of the conical bottom may vary or the bottom may have rounded or other shape. The recesses provided at the pressure element 6 may also have a different shape. As shown in Fig. 8, the recesses 610a, 610b can have, for example, a substantially rectangular cross-section. A lower side of the recess comprises an inclined edge 611a, 611b for engagement with the deformed portions 502a, 502b.

As shown in Fig. 9, the cross-section of the recesses 620a, 620b of the pressure element can be for example trapezoidal, with an inclined lower flank 621a, 621b for engagement with the deformed portions 502a, 502b.

All parts of the bone anchoring device are made of a body-compatible material, such as a body-compatible metal, for example, titanium, body-compatible metal alloys such as, for example, Nitinol or from a body-compatible plastic material, such as, for example, polyether ether ketone (PEEK) or combinations thereof.

Usually, several bone anchoring devices are necessary for stabilizing bone parts or vertebrae with the rod. In use, the bone anchoring devices are pre-assembled as shown in Fig. 5a, 5b. The screw members are screwed into the bone or a vertebra. Then, the receiving parts are pivoted by applying a force greater than the friction force until each receiving part has the correct orientation for the insertion of the rod. Due to the friction force, each receiving part is held in this angular position. Thereafter, the rod, which connects the bone anchoring devices, is inserted and the inner screw is tightened to move the pressure element downwards to lock the head in the seat so that the angular position of the screw member with respect to the receiving part is fixed. The rod is simultaneously fixed by the inner screw. Since the deformed portions 502a, 502b engage only the lower flank of the recesses provided at the pressure element, the recesses provide enough space for the deformed portions to allow a downward movement of the pressure element.

Further modifications of the previously described embodiment are conceivable. For example, only one deformed portion at the receiving part and one corresponding recess at the pressure element is sufficient. However, more than two deformed portions and corresponding recesses can also be provided.

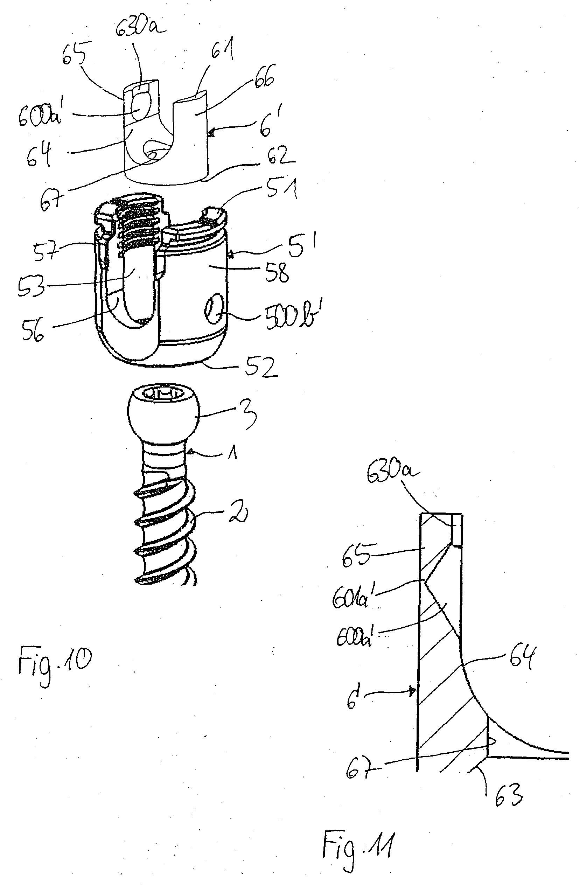

A second embodiment of the bone anchoring device is described with reference to Figs. 10 to 13. Parts or portions that are identical or similar to the previously decribed embodiment are designated with the same reference numerals and the decription thereof will not be repeated. The second embodiment differs from the first embodiment mainly in that the functions of the pressure element and the receiving part with respect to the provisional fixation with preload onto the head are reversed.

As can be seen in Fig. 9, the receiving part 5' has instead of the blind holes 500a, 500b two through holes 500a', 500b'. Although recesses at the inner wall of the receiving part instead of through holes would be sufficient, providing through holes is easier to manufacture and allows to upgrade existing receiving parts that have the blind holes of the first embodiment.

The pressure element 6' has two recesses 600a', 600b', arranged offset by 180°, that extend from the inner wall of the channel 64 into the legs 65, 66, respectively. The recesses can have a substantially triangular cross-section with a taper of approximately 22,5° similar to that of the blind holes 500a, 500b of the receiving part of the first embodiment. At the upper edge of the recesses a rectangular recess 630a, 630b is provided, respectively, the depth of which is smaller than that of the recesses 600a', 600b'. The recesses 630a, 630b are optional and may facilitate the insertion of a crimping tool.

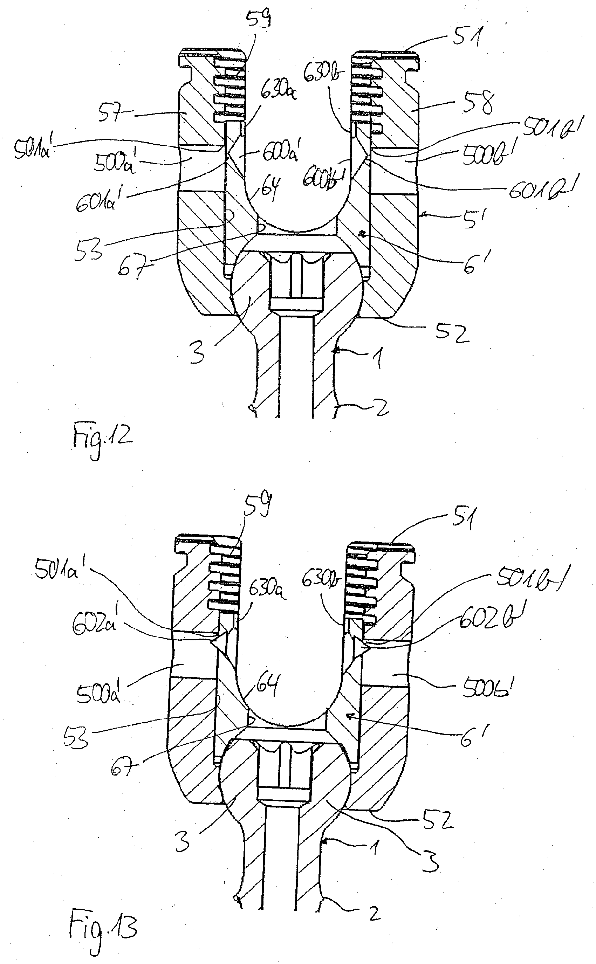

Between the outer surface of the pressure element 6' and the bottom of the recesses 600a', 600b', deformable portions 601 a', 601b' are formed that can be deformed into deformed portions 602a', 602b' as shown in Fig. 13. In the pre-assembled and non-deformed state, as shown in Fig. 12, the pressure element 6' is situated in the receiving part 5' in such a position that it rests on the head 4 and the deformable portions are slightly below the upper wall portion of the through holes 500a', 500b'. Then, crimping tips (not shown) are introduced into the recesses 600a', 600b' and the deformable portions 601a', 601b' are deformed towards the outside. the deformed portions 602a', 602b' abut against the upper wall portion (501a', 501b') of the through holes 500a', 500b' at the inner side of the receiving part 5' as shown in Fig. 13. The deformed portions 602a', 602b' have then a taper of around 45°. When the deformed portions abut against the upper wall portion (501a', 501b') of the through holes, a downward force is exerted onto the head which clamps the head by friction.

The shape of the recesses and blind holes of the embodiments described is not limited to the tapered form. Also the angles of the taper are not limited to the values described. Other shapes are possible that achieve also a downwardly directed force when the deformable portions are deformed.

For the anchoring element, all kinds of anchoring elements can be used and combined with a receiving part. These anchoring elements are e.g. screws of different lengths, with different diameters, cannulated screws, screws with different thread forms, nails, hooks, etc. The head and the shaft can be separate parts which are connectable to each other.

The shape of the receiving part is not limited to the embodiment shown. For example, the receiving part can have an asymmetric end portion for allowing a greater pivot angle of the screw member to one side. The seat for the head may be provided in an insert piece being part of the receiving part. Also, it is possible to have a recess allowing the rod to be introduced from the side instead of being introduced from the top or a closed recess through which the rod has to be guided. Various kinds of locking devices, including two- or more-part locking devices, outer nuts, outer caps, bayonet locking devices or others are possible.

In a further modification, the receiving part is configured to allow the introduction of the screw element from the bottom end.

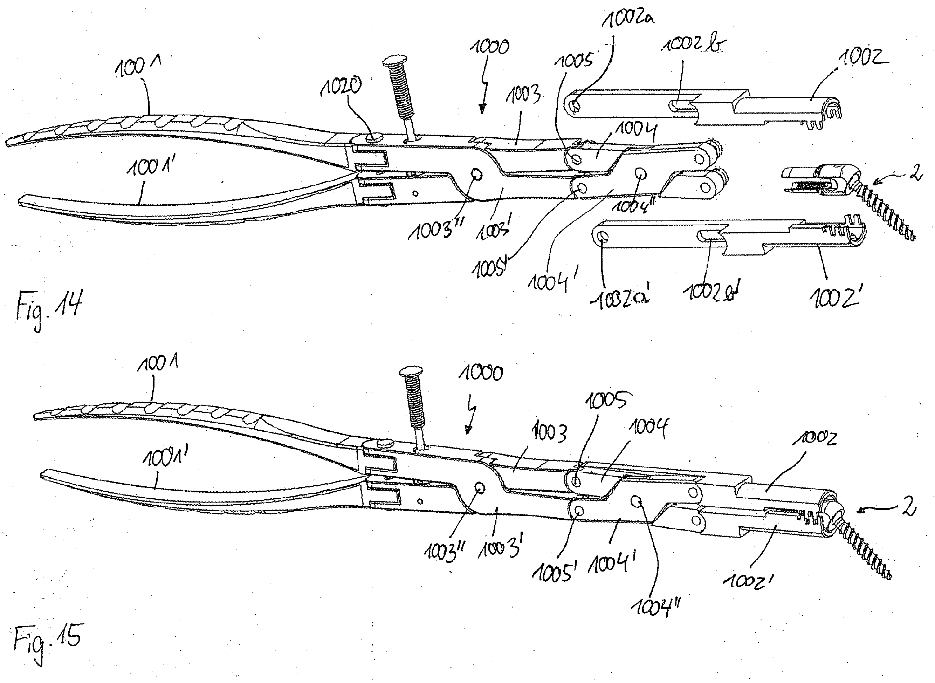

Figs. 14 and 15 show a further embodiment of a crimping tool to be used for manufacturing the polyaxial bone anchoring device. The crimping tool 1000 can be applied to enhance the friction force between the pressure element 6 and the screw head 4 of the pre-assembled bone anchoring device. As can be gathered from Figs. 14 and 15, the crimping tool 1000 is implemented as a hand-held instrument in the form of tongs having a pair of handles 1001, 1001' at one end and a pair of claws 1002, 1002' at the opposite end. Between the handles 1001, 1001' and the claws 1002, 1002' two pairs of levers are arranged. A first pair of levers 1003, 1003' that pivot around pivot 1003" are connected at one side to the handles 1001, 1001' and at the other side to a second pair of levers 1004, 1004' that pivot around pivot 1004". The pair of levers 1004, 1004' is hingedly connected to the pair of levers 1003, 1003' at pivots 1005, 1005' and is connected at its free end to the claws 1002, 1002'. The claws 1002, 1002' are fixed at their rear end 1002a, 1002a' at the respective lever arm of the lever 1004, 1004' that is connected to the lever arm 1003, 1003'. The other lever arm of the lever 1004, 1004' is connected via an elongate hole 1002, 1002b', 1002b to the respective other claw 1002', 1002. With this construction, the moving of the handles 1001, 1001' together causes the claws 1002, 1002' to move towards each other thereby being aligned substantially parallel. The crimping tool 1000 may include a spring (not shown) that holds the claws 1002, 1002' in an open position and the spring force of which must be overcome by the handles to close the claws 1002, 1002'. The handles 1001, 1001' are shown to be in the same direction as the series of levers and the claws. However, handles 1001, 1001' may be rotated at a hinge 1020 so that they are perpendicular to the claws 1002, 1002'. This may be of advantage in certain cases. It shall be noted that the crimping tool can be realized by any other tongs-like construction in which a pressing of the handles together causes the claws to move towards each other.

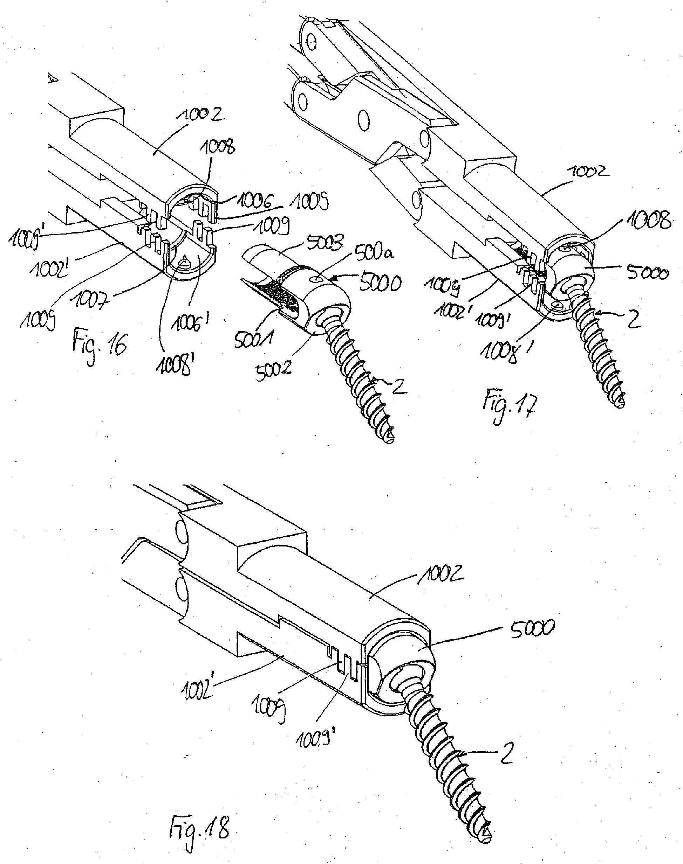

As shown in more detail in Fig. 16, the claws 1002, 1002' each have a recess 1006, 1006' that can accommodate the receiving part 5 of the polyaxial bone anchoring device in a positive-fit manner. At the end of the recess 1006, 1006' in an axial direction along the axis of the crimping tool 1000 a stop 1007, 1007' is provided against which an upper end portion 5001 of the receiving part 5000 abuts when the receiving part is inserted into one of the claws 1002, 1002'. The receiving part 5000 includes an asymmetric inclined lower end 5002 for providing a greater pivot angle to one side and an extension 5003 that can be broken off later. This shape is only an example and the tool is applicable to any other receiving part.

At the inner wall of each recess 1006, 1006' crimping tips 1008, 1008' are provided. The crimping tips 1008, 1008' are located at a position corresponding to the central axis A, B of the blind-holes 500a, 500b of the receiving part 5000. Hence, when the polyaxial bone anchoring device is inserted and abuts against the stop 1007, the crimping tips 1008, 1008' point in the direction of the central axis A, B of the blind-holes 500a, 500b.

At both sides of each recess 1006, 1006' the claws 1002 comprise a teeth 1009, 1009'. The teeth 1009 of one claw 1002 engage the space between the teeth 1009' of the other claw 1002' when the claws 1002, 1002' move together until they are closed around the receiving part 5000 as shown in Fig. 18.

The operation will now be described with reference to Figs. 16 to 20. The bone anchoring device 5000 can be delivered, as described before, in the pre-assembled state with the screw element 2 being inserted and the pressure element 6 being held by crimping in such a way that its U-shaped recess 64 is aligned with the U-shaped recess 56 of the receiving part 5000. By means of the deformed portions 502a, 502b that protrude into the recesses 600a, 600b of the pressure element, the pressure element 6 exerts a preload onto the head 4 to frictionally hold the head 4 in a certain angular position.

An additional crimping step can be applied in a case in which the friction force between the pressure element 6 and the head 4 of the pre-assembled polyaxial bone anchoring device is too low. By the additional crimping step , the surgeon or any other assistant personnel may produce a polyaxial bone anchoring device with a high friction force between the pressure element 6 and the head 4. This can be done at any time before or during surgery. The additional crimping is carried out, before the screw element is screwed into the bone. Before surgery, any other crimping tool or the crimping tool 1000 can be used for performing the additional crimping step.

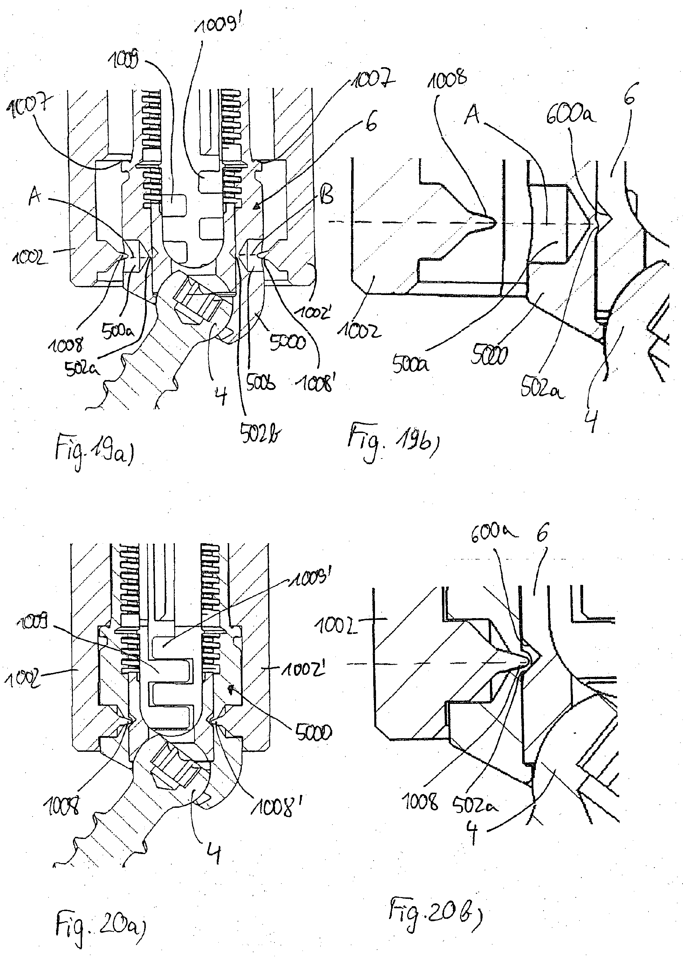

Using the crimping tool 1000 as shown in the Figures, in a first step, the polyaxial bone anchoring device is inserted between the claws 1002, 1002' of the crimping tool 1000 such that the blind-holes 500a, 500b are aligned with the crimping tips 1008, 1008'. Since the stop 1007, 1007' is provided, the crimping tips 1008, 1008'are automatically at the correct position. As shown in Fig. 19a) and 19b), when the claws 1002, 1002' are open, the upper end 1001 of the receiving part 5000 abuts against the stop 1007, 1007' preventing further insertion of the bone anchoring device. The central axes of the crimping tips 1008, 1008' is coaxial with the central axis A, B of the blind-holes 500a, 500b, respectively.

Thereafter, the handles 1001, 1001' are pressed together, thereby closing the claws 1002, 1002' as shown in Figs. 20a) and 20b). The crimping tips 1008, 1008' are moved in the direction of the deformed portions 502a, 502b and further deform the deformed portions 502a, 502b so that they extend further into the recesses 600a, 600b of the pressure element 6 and exert a downward pressure force onto the pressure element. This leads to an increase of the pre-load exerted by the pressure element 6 onto the head 4, resulting in a higher friction between the pressure element and the head. The movement of the crimping tips is limited by the form-fit engagement of the inner contour of the recesses 1006, 1006' with the outer contour of the receiving part 5000. Since this engagement provides a stop, the crimping tips 1008, 1008'move along a path with a defined length to generate a defined deformation to enhance the friction force.
The claws 1002, 1002' engage each other in a form-fit connection when the teeth 1009' of the lower claw 1002' engage the teeth 1009 of the upper claw 1002.

The crimping tool can reliably produce a certain high friction force between the pressure element 6 and the head 4.

According to an aspect a polyaxial bone anchoring device is provided including an anchoring element 2 having a shaft 3 for anchoring in a bone and a head 4, a receiving part 5, 5' having a top end 51 and a bottom end 52, a channel 56 for receiving a rod therein and a passage 53, 54, 55 extending from the top end 51 to the bottom end 52 and including a seat 54 for receiving the head 4, a pressure element 6 which is arranged in the passage 53 and configured to exert pressure onto the head 4, wherein the head is pivotable with respect to the receiving part 5 and can be locked at an angle by means of the pressure element 6, the pressure element 6, 6' comprising at least one recess 600a, 600b; 610a, 610b; 620a, 620b facing the inner wall of the receiving part or a deformable portion 601a', 601b' at its outer wall, the receiving part 5 including at least one deformable portion 501a, 501b provided at its inner wall, which upon deformation engages the recess 600a, 600b; 610a, 610b; 620a, 620b at the pressure element or a recess 500a', 500b' open at least to its inner wall, wherein the deformable portion 502a, 502b; 601a', 601b' and the recess 600a, 600b; 610a, 610b; 620a, 620b; 500a', 500b' are arranged such that by engagement the deformable portion with the recess a force is exerted by the pressure element 6 onto the head that maintains the head at an angular position by friction before locking the head.

According to a further aspect the deformable portion 501a, 501b, 601a', 601b' is configured to plastically deform into a deformed portion 502a, 502b, 602a', 602b' extending into the passage 53.

According to a further aspect the pressure element 6 is inserted into the receiving part 5 and rests upon the head 4, the recess 600a, 600b has a lower edge 601 a, 601 b, 611a, 611b; 621 a, 621b that is located above the deformable portion 501a, 501b in an axial direction towards the first end 51.

According to a further aspect the distance in axial direction between the lower edge of the recess and the deformable portion 501 a, 501b in such that the deformed portion exerts a pressure onto the pressure element, when engaging the lower edge 601 a, 601 b; 611 a, 611 b; 621 a, 621 b of the recess to generate a defined friction force between the head and the pressure element. According to a further aspect the receiving part includes a blind hole 500a, 500b extending from the outer wall of the receiving part towards the inner wall and wherein the deformable portion 501a, 501b is arranged between the inner wall and the blind hole.

According to a further aspect the deformable portion (501a, 501b) is deformable into a tapered protrusion extending from the inner wall of the receiving part into the passage.

According to a further aspect a closed end of the blind hole is tapered and includes an angle (β) with the central axis (M) of the passage of less than 45°, preferably of around 22,5°.

According to a further aspect the recess (600a, 600b; 610a, 610b; 620a, 620b) has an inclined lower edge (601a, 611 a, 621a), which includes an angle with the central axis (M) of the passage of preferably around 45°.

According to a further aspect the recesses (600a, 600b) provide space for the deformed portion (502a, 502b) in an axial direction towards the first end (51).

According to a further aspect at least two deformable portions (501a, 501b) and corresponding recesses (600a, 600b; 610a, 610b; 620a, 620b) are provided, which are offset by 180° in a circumferential direction.

According to a further aspect the deformable portion (601a', 601b') is provided at the pressure element (6') which upon deformation engages an upper edge (501a', 501b') of the recess (500a', 500b') provided at the receiving part (5').

According to a further aspect the pressure element 6, 6' includes a top end 61 and a bottom end 62, a coaxial bore 67, a spherical recess 63 at the bottom end and a cylindrical or U-shaped recess 64 at the top end and wherein the cylindrical or U-shaped recess 64 is aligned with the recess 56 of the receiving part.

According to a further aspect a polyaxial bone anchoring device is provided including an anchoring element 2 having a shat(3 for anchoring in a bone and a head 4, a receiving part 5 having a top end 51 and a bottom end 52, a channel 56 for receiving a rod therein and a passage 53, 54, 55 extending from the top end 51 to the bottom end 52 and including a seat 54 for receiving the head 4, a pressure element 6 which is arranged in the passage 53 and configured to exert pressure onto the head 4, wherein the head is pivotable with respect to the receiving part 5 and can be locked at an angle by means of the pressure element 6, the pressure element 6 comprising at least one recess 600a, 600b; 610a, 610b; 620a, 620b or a protrusion 602a', 602b' facing the inner wall of the receiving part, the receiving part comprising at least one protrusion 502a, 502b or recess 500a', 500b' provided at its inner wall, wherein, when the pressure element is inserted into the receiving part and rests on the head, the protrusion 502a, 502b, 602a', 602b' engages an edge 601a, 601b; 611a, 611b; 621a, 621 b, 501a', 501b' of the recess such that by the engagement a force is exerted by the pressure element onto the head 4 to maintain the head at an angular position by friction before locking the head.

According to a further aspect at least two protrusions 502a, 502b, 602a', 602b' and corresponding recesses 600a, 600b; 610a, 610b; 620a, 620b, 500a', 500b' are provided, which are offset by 180°.

## Claims

1. A method for manufacturing a polyaxial bone anchoring device, wherein the polyaxial bone anchoring device includes
an anchoring element (2) having a shaft (3) for anchoring in a bone and a head (4); a receiving part (5, 5') having a top end (51) and a bottom end (52), a channel (56) for receiving a rod therein and a passage (53, 54, 55) extending from the top end (51) to the bottom end (52) and including a seat (54) for receiving the head (4);
a pressure element (6) which is arranged in the passage (53) and configured to exert pressure onto the head (4), wherein the pressure element is a solid member without a spring portion and wherein the head is pivotable with respect to the receiving part (5) and can be locked at an angle by means of the pressure element (6);
the pressure element (6, 6') comprising at least one recess (600a, 600b; 610a, 610b; 620a, 620b) facing the inner wall of the receiving part or a deformable portion (601a', 601 b') at its outer wall;
the receiving part (5) including at least one deformable portion (501 a, 501b) provided at its inner wall, which upon deformation engages the recess (600a, 600b; 610a, 610b; 620a, 620b) at the pressure element or a recess (500a', 500b') open at least to its inner wall;
wherein the deformable portion (502a, 502b; 601a', 601b') and the recess (600a, 600b; 610a, 610b; 620a, 620b; 500a', 500b') are arranged such that by engagement the deformable portion with the recess a force is exerted by the pressure element (6) onto the head that maintains the head at an angular position by friction before locking the head,
wherein the method includes the steps
arranging the pressure element (6, 6') in the receiving part (5, 5') such that the recess (600a, 600b; 610a, 610b; 620a, 620b, 500a', 500b') is located with respect to the deformable portion (501a, 501b, 601a', 601b') in an axial direction in such a way that the reformable portion (501a, 501b, 601a', 601b') can engage upon deformation an edge (601a, 601b, 501a', 501b') of the recess;
deforming the deformable portion (501a, 501b, 601a', 601b') such that the deformed portion (502a, 502b, 602a', 602b') forms a protrusions, which engages the edge (601a, 611a, 621a, 501a', 501b') of the recess, thereby exerting a force onto the pressure element to maintain the head in an angular position by friction before locking it, wherein the step of deforming is carried out before the bone anchoring element is inserted into the bone.

2. A method for manufacturing a polyaxial bone anchoring device, wherein the polyaxial bone anchoring device includes
an anchoring element (2) having a shaft (3) for anchoring in a bone and a head (4);
a receiving part (5) having a top end (51) and a bottom end (52), a channel (56) for receiving a rod therein and a passage (53, 54, 55) extending from the top end (51) to the bottom end (52) and including a seat (54) for receiving the head (4);
a pressure element (6) which is arranged in the passage (53) and configured to exert pressure onto the head (4), wherein the pressure element is a solid member without a spring portion and wherein the head is pivotable with respect to the receiving part (5) and can be locked at an angle by means of the pressure element (6);
the pressure element (6) comprising at least one recess (600a, 600b; 610a, 610b; 620a, 620b) or a protrusion (602a', 602b') facing the inner wall of the receiving part;
the receiving part comprising at least one protrusion (502a, 502b) or recess (500a', 500b') provided at its inner wall, wherein, when the pressure element is inserted into the receiving part and rests on the head, the protrusion (502a, 502b, 602a', 602b') engages an edge (601 a, 601b; 611a, 611b; 621 a, 621b, 501a', 501b') of the recess such that by the engagement a force is exerted by the pressure element onto the head (4) to maintain the head at an angular position by friction before locking the head,
wherein the method includes the steps
arranging the pressure element (6, 6') in the receiving part (5, 5') such that the recess (600a, 600b; 610a, 610b; 620a, 620b, 500a', 500b') is located with respect to a deformable portion (501a, 501b, 601a', 601b') in an axial direction in such a way that the deformable portion (501a, 501b, 601a', 601b') can engage upon deformation an edge (601a, 601b, 501a', 501b') of the recess;
deforming the deformable portion (501a, 501b, 601a', 601b') such that the deformed portion (502a, 502b, 602a', 602b') forms the protrusions, which engages the edge (601a, 611a, 621a, 501a', 501b') of the recess, thereby exerting a force onto the pressure element to maintain the head in an angular position by friction before locking it, wherein the step of deforming is carried out before the bone anchoring element is inserted into the bone.

3. The method of claim 1 or 2, wherein the recess (600a, 600b) is formed at the pressure element (6) and the protrusion is formed at the receiving part (5) and wherein the protrusion is located above a lower edge (601a, 601b) of the recess.

4. The method of claim 1 or 2, wherein the recess (500a', 500b') is formed at the receiving part (5') and the protrusion is formed at the pressure element (6') and wherein the protrusion is located below an upper edge (501a', 501b') of the recess.

5. The method of one of claims 1 to 4, wherein the deformation is made by crimping.

6. The method of one of claims 1 to 5, wherein the deformation of the deformable portions (501a, 501b, 601a', 601b') is generated using a conventional crimping tool (100).

7. The method of one claims 1 to 6, wherein the deformation is made using a hand-held crimping tool (1000).

8. The method of one of claims 1 to 7, wherein in a first step the deformation of the deformable portions (501a, 501b, 601a', 601b') is made during pre-assembly and wherein after pre-assembly an additional deformation is made.

9. The method according to one of claims 1 to 8 using a tool comprising tongs (1000) with ends in form of handles (1001, 1001') and
claws (1002, 1002') for gripping a receiving part (5, 5000) of a polyaxial bone anchoring device,
wherein the claws (1002, 1002') comprise at least one crimping tip (1008, 1008')

10. The method of claim 9, wherein the path of movement of the crimping tips (1008, 1008') is limited by a stop.

11. The method of claim 9 or 10, wherein at least one of the claws (1002, 1002') provides a stop (1007, 1007') to limit the insertion of the receiving part (5000).

## Patentansprüche

1. Verfahren zum Herstellen einer polyaxialen Knochenverankerungsvorrichtung, wobei die polyaxiale Knochenverankerungsvorrichtung umfasst
ein Verankerungselement (2) mit einem Schaft (3) zum Verankern in einem Knochen und einen Kopf (4);
ein Aufnahmeteil (5, 5') mit einem oberen Ende (51) und einem unterem Ende (52), einem Kanal (56) zur Aufnahme eines Stabes darin und einen Durchlass (53, 54, 55), welcher sich von dem oberen Ende (51) zu dem unteren Ende (52) erstreckt und einen Sitz (54) zum Aufnehmen des Kopfes (4) umfasst;
ein Druckelement (6) welches in dem Durchlass (53) angeordnet ist und ausgebildet ist Druck auf den Kopf (4) auszuüben, wobei das Druckelement ein festes Teil ohne einen Federabschnitt ist und wobei der Kopf in Bezug auf das Aufnahmeteil (5) schwenkbar ist und mittels des Druckelements (6) unter einem Winkel verriegelt werden kann;
wobei das Druckelement (6, 6') zumindest eine Ausnehmung (600a, 600b; 610a, 610b; 620a, 620b) aufweist, welche der Innenwand des Aufnahmeteils zugewandt ist, oder einen deformierbaren Abschnitt (601a', 601b') an seiner Außenwand;
wobei das Aufnahmeteil (5) zumindest einen deformierbaren Abschnitt (501a, 501b) umfasst, welcher an seiner Innenwand vorgesehen ist und bei Deformation mit der Ausnehmung (600a, 600b; 610a, 610b; 620a, 620b) in dem Druckelement in Eingriff gelangt, oder eine Ausnehmung (500a', 500b') welche zumindest zu seiner Innenwand hin offen ist;
wobei der deformierbare Abschnitt (502a, 502b; 601a', 601b') und die Ausnehmung (600a, 600b; 610a, 610b; 620a, 620b; 500a', 500b') so angeordnet sind, dass durch in-Eingriff-Bringen des deformierbaren Abschnitts mit der Ausnehmung eine Kraft durch das Druckelement (6) auf den Kopf ausgeübt wird, welche den Kopf vor Verriegeln des Kopfes durch Reibung in einer Winkelstellung hält,
wobei das Verfahren die Schritte umfasst
Anordnen des Druckelements (6, 6') in dem Aufnahmeteil (5, 5') so dass die Ausnehmung (600a, 600b; 610a, 610b; 620a, 620b; 500a', 500b') in Bezug auf den deformierbaren Abschnitt (501a, 501b, 601a', 601b') in einer axialen Richtung so gelegen ist, dass der deformierbare Abschnitt (501a, 501b, 601a', 601b') bei Deformierung mit einer Kante (601a, 601b, 501a', 501b') der Ausnehmung in Eingriff gelangen kann;
Deformieren des deformierbaren Abschnitts (501a, 501b, 601a', 601b') so dass der deformierte Abschnitt (502a, 502b, 602a', 602b') einen Vorsprung bildet, welcher mit der Kante (601a, 611a, 621a, 501a', 501b') der Ausnehmung in Eingriff gelangt, dabei eine Kraft auf das Druckelement ausübt um den Kopf vor Verriegeln des Kopfes durch Reibung in einer Winkelstellung zu halten, wobei der Schritt des Deformierens ausgeführt wird bevor das Knochenverankerungselement in den Knochen eingesetzt wird.

2. Verfahren zum Herstellen einer polyaxialen Knochenverankerungsvorrichtung, wobei die polyaxiale Knochenverankerungsvorrichtung umfasst
ein Verankerungselement (2) mit einem Schaft (3) zum Verankern in einem Knochen und einen Kopf (4);
ein Aufnahmeteil (5) mit einem oberen Ende (51) und einem unterem Ende (52), einem Kanal (56) zur Aufnahme eines Stabes darin und einen Durchlass (53, 54, 55), welcher sich von dem oberen Ende (51) zu dem unteren Ende (52) erstreckt und einen Sitz (54) zum Aufnehmen des Kopfes (4) umfasst;
ein Druckelement (6) welches in dem Durchlass (53) angeordnet ist und ausgebildet ist Druck auf den Kopf (4) auszuüben, wobei das Druckelement ein festes Teil ohne einen Federabschnitt ist und wobei der Kopf in Bezug auf das Aufnahmeteil (5) schwenkbar ist und mittels des Druckelements (6) unter einem Winkel verriegelt werden kann;
wobei das Druckelement (6) zumindest eine Ausnehmung (600a, 600b; 610a, 610b; 620a, 620b) oder einen Vorsprung (602a', 602b') aufweist, welche oder welcher der Innenwand des Aufnahmeteils zugewandt ist;
wobei das Aufnahmeteil zumindest einen Vorsprung (502a, 502b) oder eine Ausnehmung (500a', 500b') an seiner Innenwand aufweist, wobei, wenn das Druckelement in das Aufnahmeteil eingesetzt ist und auf dem Kopf aufliegt, der Vorsprung (502a, 502b, 602a', 602b') mit einer Kante (601a, 601b; 611a, 611b; 621a, 621b, 501a', 501b') der Ausnehmung in Eingriff gelangt, so dass durch das in-Eingriff-Bringen eine Kraft durch das Druckelement (6) auf den Kopf ausgeübt wird um den Kopf vor Verriegeln des Kopfes durch Reibung in einer Winkelstellung zu halten,
wobei das Verfahren die Schritte umfasst
Anordnen des Druckelements (6, 6') in dem Aufnahmeteil (5, 5') so dass die Ausnehmung (600a, 600b; 610a, 610b; 620a, 620b; 500a', 500b') in Bezug auf einen deformierbaren Abschnitt (501a, 501b, 601a', 601b') in einer axialen Richtung so gelegen ist, dass der deformierbare Abschnitt (501a, 501b, 601a', 601b') bei Deformierung mit einer Kante (601a, 601b, 501a', 501b') der Ausnehmung in Eingriff gelangen kann;
Deformieren des deformierbaren Abschnitts (501a, 501b, 601a', 601b') so dass der deformierte Abschnitt (502a, 502b, 602a', 602b') den Vorsprung bildet, welcher mit der Kante (601a, 611a, 621a, 501a', 501b') der Ausnehmung in Eingriff gelangt, dabei eine Kraft auf das Druckelement ausübt um den Kopf vor Verriegeln des Kopfes durch Reibung in einer Winkelstellung zu halten, wobei der Schritt des Deformierens ausgeführt wird bevor das Knochenverankerungselement in den Knochen eingesetzt wird.

3. Verfahren nach Anspruch 1 oder 2, wobei die Ausnehmung (600a, 600b) an dem Druckelement (6) gebildet ist und der Vorsprung an dem Aufnahmeteil (5) gebildet ist und wobei der Vorsprung über einer unteren Kante (601a, 601b) der Ausnehmung gelegen ist.

4. Verfahren nach Anspruch 1 oder 2, wobei die Ausnehmung (500a', 500b') an dem Aufnahmeteil (5') gebildet ist und der Vorsprung an dem Druckelement (6') gebildet ist und wobei der Vorsprung unter einer oberen Kante (501a', 501b') der Ausnehmung gelegen ist.

5. Verfahren nach einem der Ansprüche 1 bis 4, wobei die Deformation durch Crimpen verursacht wird.

6. Verfahren nach einem der Ansprüche 1 bis 5, wobei die Deformation des deformierbaren Abschnitts (501a, 501b, 601a', 601b') durch Verwenden eines herkömmlichen Crimpwerkzeuges (100) hervorgerufen wird.

7. Verfahren nach einem der Ansprüche 1 bis 6, wobei die Deformation durch Verwenden eines in der Hand gehaltenen Crimpwerkzeuges (1000) verursacht wird.

8. Verfahren nach einem der Ansprüche 1 bis 7, wobei in einem ersten Schritt die Deformation des deformierbaren Abschnitts (501a, 501b, 601a', 601b') während des Vormontierens durchgeführt wird und wobei nach dem Vormontieren eine zusätzliche Deformation durchgeführt wird.

9. Verfahren nach einem der Ansprüche 1 bis 8, wobei ein Werkzeug verwendet wird, welches Zangen (1000) mit Enden in Form von Griffen (1001, 1001') umfasst und
Greifer (1002, 1002') zum Greifen eines Aufnahmeteils (5, 5000) einer polyaxialen Knochenverankerungsvorrichtung,
wobei die Greifer (1002, 1002') zumindest eine Crimpspitze (1008, 1008') umfassen.

10. Verfahren nach Anspruch 9, wobei der Bewegungsweg der Crimpspitzen (1008, 1008') durch einen Anschlag begrenzt ist.

11. Verfahren nach Anspruch 9 oder 10, wobei zumindest einer der Greifer (1002, 1002') einen Anschlag aufweist um das Einbringen des Aufnahmeteils (5000) zu beschränken.

## Revendications

1. Procédé pour la fabrication d'un dispositif d'ancrage d'os polyaxial, où le dispositif d'ancrage d'os polyaxial comprend
un élément (2) d'ancrage pourvu d'une tige (3) destinée à être ancrée dans un os et d'une tête (4) ;
une pièce (5, 5') de réception présentant une extrémité (51) supérieure et une extrémité (52) inférieure, un canal (56) destiné à recevoir une barre à l'intérieur de ce dernier et un passage (53, 54, 55) s'étendant de l'extrémité (51) supérieure à l'extrémité (52) inférieure et comprenant un siège (54) destiné à recevoir la tête (4) ;
un élément (6) de pression qui est agencé dans le passage (53) et configuré pour exercer une pression sur la tête (4), où l'élément de pression est un élément plein sans partie ressort et où la tête peut pivoter par rapport à la pièce (5) de réception et peut être verrouillée à un angle au moyen de l'élément (6) de pression ;
l'élément (6, 6') de pression comportant au moins un évidement (600a, 600b ; 610a, 610b ; 620a, 620b) faisant face à la paroi interne de la pièce de réception ou une partie (601a', 601b') déformable au niveau de sa paroi externe ;
la pièce (5) de réception comprenant au moins une partie (501a, 501b) déformable fournie au niveau de sa paroi interne, qui, au moment de la déformation, vient en contact avec l'évidement (600a, 600b ; 610a, 610b ; 620a, 620b) au niveau de l'élément de pression ou d'un évidement (500a', 500b') ouvert au moins vers sa paroi interne ;
où la partie (502a, 502b ; 601a', 601b') déformable et l'évidement (600a, 600b ; 610a, 610b ; 620a, 620b ; 500a', 500b') sont agencés de sorte que, par le contact de la partie déformable avec l'évidement, une force est exercée par l'élément (6) de pression sur la tête qui maintient la tête à une position angulaire par frottement avant de verrouiller la tête,
où le procédé comprend les étapes consistant à agencer l'élément (6, 6') de pression dans la pièce (5, 5') de réception de sorte que l'évidement (600a, 600b ; 610a, 610b ; 620a, 620b, 500a', 500b') est situé par rapport à la partie (501a, 501b, 601a', 601b') déformable dans une direction axiale de telle manière que la partie (501a, 501b, 601a', 601b') déformable puisse, au moment de la déformation, venir en contact avec un bord (601a, 601b, 501a', 501b') de l'évidement ;
déformer la partie (501a, 501b, 601a', 601b') déformable de sorte que la partie (502a, 502b, 602a', 602b') déformée forme une protubérance, qui vient en contact avec le bord (601a, 611a, 621a, 501a', 501b') de l'évidement, exerçant ainsi une force sur un élément de pression pour maintenir la tête dans une position angulaire par frottement avant de la verrouiller, où l'étape consistant à déformer s'effectue avant que l'élément d'ancrage d'os soit inséré dans l'os.

2. Procédé pour la fabrication d'un dispositif d'ancrage d'os polyaxial, où le dispositif d'ancrage d'os polyaxial comprend
un élément (2) d'ancrage pourvu d'une tige (3) destinée à être ancrée dans un os et d'une tête (4) ;
une pièce (5) de réception présentant une extrémité (51) supérieure et une extrémité (52) inférieure, un canal (56) destiné à recevoir une barre à l'intérieur de ce dernier et un passage (53, 54, 55) s'étendant de l'extrémité (51) supérieure à l'extrémité (52) inférieure et comprenant un siège (54) destiné à recevoir la tête (4) ;
un élément (6) de pression qui est agencé dans le passage (53) et configuré pour exercer une pression sur la tête (4), où l'élément de pression est un élément plein sans partie ressort et où la tête peut pivoter par rapport à la pièce (5) de réception et peut être verrouillée à un angle au moyen de l'élément (6) de pression ;
l'élément (6) de pression comportant au moins un évidement (600a, 600b ; 610a, 610b ; 620a, 620b) ou une protubérance (602a', 602b') faisant face à la paroi interne de la pièce de réception ;
la pièce de réception comportant au moins une protubérance (502a, 502b) ou un évidement (500a', 500b') fourni au niveau de sa paroi interne, où, lorsque l'élément de pression est inséré dans la pièce de réception et repose sur la tête, la protubérance (502a, 502b, 602a', 602b') vient en contact avec un bord (601a, 601b ; 611a, 611b ; 621a, 621b, 501a', 501b') de l'évidement de sorte que, par le contact, une force est exercée par l'élément de pression sur la tête (4) pour maintenir la tête à une position angulaire par frottement avant de verrouiller la tête,
où le procédé comprend les étapes consistant à agencer l'élément (6, 6') de pression dans la pièce (5, 5') de réception de sorte que l'évidement (600a, 600b ; 610a, 610b ; 620a, 620b, 500a', 500b') est situé par rapport à une partie (501a, 501b, 601a', 601b') déformable dans une direction axiale de telle manière que la partie (501a, 501b, 601a', 601b') déformable puisse venir en contact au moment de la déformation avec un bord (601a, 601b, 501a', 501b') de l'évidement ;
déformer la partie (501a, 501b, 601a', 601b') déformable de sorte que la partie (502a, 502b, 602a', 602b') déformée forme une protubérance, qui vient en contact avec le bord (601a, 611a, 621a, 501a', 501b') de l'évidement, exerçant ainsi une force sur l'élément de pression pour maintenir la tête dans une position angulaire par frottement avant de la verrouiller, où l'étape consistant à déformer s'effectue avant que l'élément d'ancrage d'os soit inséré dans l'os.

3. Procédé selon la revendication 1 ou 2, où l'évidement (600a, 600b) est formé au niveau de l'élément (6) de pression et la protubérance est formée au niveau de la pièce (5) de réception et où la protubérance est située au-dessus d'un bord (601a, 601b) inférieur de l'évidement.

4. Procédé selon la revendication 1 ou 2, où l'évidement (500a', 500b') est formé au niveau de la pièce (5') de réception et la protubérance est formée au niveau de l'élément (6') de pression et où la protubérance est située en dessous d'un bord (501a', 501b') supérieur de l'évidement.

5. Procédé selon l'une quelconque des revendications 1 à 4, où la déformation s'effectue par sertissage.

6. Procédé selon l'une quelconque des revendications 1 à 5, où la déformation des parties (501a, 501b, 601a', 601b') déformables est produite en utilisant un outil (100) de sertissage traditionnel.

7. Procédé selon l'une quelconque des revendications 1 à 6, où la déformation s'effectue en utilisant un outil (1000) de sertissage tenu à la main.

8. Procédé selon l'une quelconque des revendications 1 à 7, où dans une première étape a déformation des parties (501a, 501b, 601a', 601b') déformables est réalisée durant le pré-assemblage et où après le pré-assemblage une déformation supplémentaire est réalisée.

9. Procédé selon l'une quelconque des revendications 1 à 8 utilisant un outil comportant des pinces (1000) dont les extrémités sont en forme de poignées (1001, 1001') et
des griffes (1002, 1002') destinées à saisir une pièce (5, 5000) de réception d'un dispositif d'ancrage d'os polyaxial,
où les griffes (1002, 1002') comportent au moins un bout (1008, 1008') de sertissage.

10. Procédé selon la revendication 9, où le chemin de déplacement des bouts (1008, 1008') de sertissage est limité par une butée.

11. Procédé selon la revendication 9 ou 10, où au moins une des griffes (1002, 1002') fournit une butée (1007, 1007') pour limiter l'insertion de la pièce (5000) de réception.
